# EUROPEAN PATENT APPLICATION

(11) **EP 3 263 010 A1**
(43) Date of publication of application: **03.01.2018**
(21) Application number: 16755261.1
(22) Date of filing: 15.02.2016
(51) Int. Cl.: A61B 1/01

(54) **OVER-TUBE FOR ENDOSCOPE, AND MEDICAL SYSTEM**

(30) Priority: 26.02.2015 JP 2015037288
(71) Applicant: Olympus Corporation, Hachioji-shi Tokyo 192-8507 (JP)
(72) Inventor: TAKEMOTO Shotaro, Hachioji-shi Tokyo 192-8507 (JP)
(74) Representative: Schicker, Silvia
(86) International application number: PCT/JP2016/054255
(87) International publication number: WO 2016/136514

(57) **Abstract**

An endoscopic overtube includes a tube body having a first lumen (3) for insertion of an insertion part of an endoscope formed therein; a shape-changeable pinching part capable of coming into contact with an outer surface of the insertion part inside the first lumen (3) and holding the insertion part by coming into contact with the outer surface of the insertion part to position the insertion part in the first lumen (3); and a manipulation part (20) attached to the tube body. The manipulation part (20) is connected to an end portion of the tube body to be rotatable with respect to the tube body about a line parallel to a center line of the first lumen (3) as a rotational center thereof, and switches by a rotational motion between a pinched state in which the insertion part of the endoscope is positioned with respect to the first lumen (3) of the tube body by the pinching part and an open state in which the insertion part can advance, retract, and rotate in the first lumen.

## Description

### Technical Field

The present invention relates to an endoscopic overtube and a medical system.

Priority is claimed on Japanese Patent Application No. 2015-037288, filed February 26, 2015, the content of which is incorporated herein by reference.

### Background Art

Conventionally, an overtube used together with an endoscope is known.

For example, Patent Literature 1 discloses an overtube which holds an insertion part of an endoscope by a pressing force or the like of an inflated balloon.

Patent Literature 2 discloses a configuration which adjusts orientation of a treatment tool by adjusting a size of a balloon disposed to abut against the treatment tool. The balloon disclosed in Patent Literature 2 is provided in a conduit for passing the treatment tool, in an endoscope cover (an endoscopic overtube) provided with a conduit for passing the endoscope and the conduit for passing the treatment tool.

An endoscopic overtube used together with an endoscope provided with a flexible insertion part has a flexible configuration so that it can be bent similarly to a curved deformation of the insertion part. Further, an endoscopic overtube used together with an endoscope to be inserted into the digestive tract can be flexibly bent to follow a shape of the digestive tract of a patient, thereby reducing a burden on the patient under treatment.

### Citation List

### Patent Literature

Patent Literature 1: Japanese Unexamined Patent Application, First Publication No. 2011-010950
Patent Literature 2: Japanese Unexamined Patent Application, First Publication No. 2011-083487

### Summary of Invention

### Technical Problem

An endoscopic overtube is inserted into a body together with an endoscope and is used. When the endoscopic overtube is used, the insertion part of the endoscope is rotated about a center line of the insertion part of the endoscope as a rotation center or the insertion part of the endoscope is rotated integrally with the endoscopic overtube, and treatment is performed while adjusting the insertion part of the endoscope to have a suitable positional relationship with respect to a treatment target part.

An endoscopic overtube configured to be flexible may be easily deformed by a force for moving the overtube forward or backward or for rotating the overtube. In this case, even if moving the overtube with a distal end thereof inserted into a body is attempted by manipulation from outside of the body, the force may not be appropriately transmitted to the distal end thereof.

According to the technique disclosed in Patent Literature 1, by utilizing an endoscope-holding mechanism provided with a balloon, it is possible to easily switch a state in which an overtube holds an endoscope and a state in which the endoscope can move inside the overtube. However, when the aforementioned switching is necessary, it is necessary to return to the manipulation of the overtube or the endoscope after changing an expansion and contraction state of the balloon by manipulating the control device for controlling expansion and contraction of the balloon. Therefore, in the technique disclosed in the above-mentioned Patent Literature 1, it is necessary to frequently shift the overtube and the endoscope, and work at a time of switching the aforementioned states is not intuitive.

The present invention has been made in view of the above circumstances, and an object thereof is to provide an endoscopic overtube in which a manipulation of holding and releasing an endoscope by the overtube is easy and intuitive.

### Solution to Problem

According to an aspect of the present invention, an endoscopic overtube, which is usable with an endoscope having an insertion part to be inserted into a body, includes: a tube body in which a first lumen for insertion of the insertion part is formed; a shape-changeable pinching part which is capable of coming into contact with an outer surface of the insertion part inside the first lumen, the pinching part being capable of holding the insertion part by coming into contact with the outer surface of the insertion part such that the insertion part is positioned in the first lumen; and a manipulation part which is connected to an end portion of the tube body such that the manipulation part is capable of performing rotational motion of rotating with respect to the tube body about a line parallel to a center line of the first lumen as a rotational center thereof, the manipulation part switching by the rotational motion between a pinched state in which the insertion part is positioned with respect to the first lumen by the pinching part and an open state in which the insertion part is capable of advancing, retracting, and rotating in the first lumen.

The pinching part may include: a balloon which is capable of expanding and contracting, the balloon being fixed to the first lumen; a suction and exhaust conduit which communicates with an inside of the balloon, the suction and exhaust conduit extending to the manipulation part along the first lumen; a supply conduit which is provided in the manipulation part and communicates with the suction and exhaust conduit to supply gas from a pump for supplying the gas to the suction and exhaust conduit; and a discharge conduit which is provided in the manipulation part and communicates with the suction and exhaust conduit to release the gas from the suction and exhaust conduit. The manipulation part may include an exhaust-stopping mechanism which closes the discharge conduit to restrict a release of the gas from the discharge conduit when a rotation angle difference with respect to the tube body becomes equal to or greater than a predetermined angle difference.

The manipulation part may have: a fixed member in which at least a part of each of the suction and exhaust conduit, the supply conduit, and the discharge conduit is disposed, the fixed member being fixed to the end portion of the tube body; a rotation member which has the exhaust-stopping mechanism, the rotation member being connected to the fixed member such that the rotation member is capable of performing the rotational motion with respect to the fixed member; and a biasing member which biases the rotation member with respect to the fixed member such that a relative position between the fixed member and the rotation member is in a predetermined positional relationship in which the discharge conduit is in an open state.

The biasing member may have an elastic tubular member which is made of an elastic member and constitutes the discharge conduit. The rotation member may have a pressing surface which closes the discharge conduit by compressing the elastic tubular member when an angle difference with respect to the fixed member equal to or larger than the predetermined angle difference is generated.

The biasing member may be made of a spring material which connects the fixed member and the rotation member.

The balloon may be disposed on an end side opposite to the end portion of the tube body.

The balloon may have a second balloon disposed at the end portion of the tube body.

The pinching part may include: an annular first airtight sealing part which is disposed at an end opposite to the end portion of the tube body in a center line direction of the tube body, the first airtight sealing part being capable of coming into contact with an outer circumferential surface of the insertion part over an entire circumference thereof; an annular second airtight sealing part which is disposed at the end portion of the tube body in the center line direction, the second airtight sealing part being capable of coming into contact with the outer circumferential surface of the insertion part over the entire circumference; a suction conduit which is opened to an inner surface of the first lumen between the first airtight sealing part and the second airtight sealing part, the suction conduit being connectable to a pump which suctions gas; and an outside air introduction conduit which is connected to the suction conduit at a part of an intermediate portion of the suction conduit. The manipulation part may include an inflow-stopping mechanism which closes the outside air introduction conduit to restrict inflow of the gas from the outside air introduction conduit when a rotation angle difference with respect to the tube body equal to or larger than a predetermined angle difference is generated.

The tube body may have a second lumen extending to be parallel to the first lumen.

According to another aspect of the present invention, a medical system includes: the endoscopic overtube according to the above aspect; an endoscope having an insertion part which is insertable into the first lumen; and the pump.

### Advantageous Effects of Invention

According to the present invention, it is possible to provide an endoscopic overtube in which a manipulation of holding and releasing the endoscope by the endoscopic overtube is easy and intuitive.

### Brief Description of Drawings

Fig. 1 is an overall view representing a medical system including an endoscopic overtube according to a first embodiment of the present invention.
Fig. 2 is a perspective view of the endoscopic overtube.
Fig. 3 is a cross-sectional view of the endoscopic overtube.
Fig. 4 is a perspective view of a distal end portion of the endoscopic overtube.
Fig. 5 is a rear view of a manipulation part of the endoscopic overtube.
Fig. 6 is a cross-sectional view taken along line A-A in Fig. 3.
Fig. 7 is a view explaining an operation of the endoscopic overtube.
Fig. 8 is a view explaining the operation of the endoscopic overtube.
Fig. 9 is a cross-sectional view representing a configuration of a modified example of the embodiment.
Fig. 10 is a cross-sectional view representing a configuration of another modified example of the embodiment.
Fig. 11 is a cross-sectional view representing an endoscopic overtube according to a second embodiment of the present invention.
Fig. 12 is a plan view representing an endoscopic overtube according to a third embodiment of the present invention.
Fig. 13 is a cross-sectional view taken along line B-B of Fig. 12.
Fig. 14 is a perspective view representing a part of a manipulation part of the endoscopic overtube.
Fig. 15 is a partial cross-sectional view representing a part of the manipulation part of the endoscopic overtube.
Fig. 16 is a schematic view explaining an operation of the endoscopic overtube.
Fig. 17 is a schematic view explaining the operation of the endoscopic overtube.
Fig. 18 is a perspective view representing an endoscopic overtube according to a fourth embodiment of the present invention.
Fig. 19 is a cross-sectional view representing an endoscopic overtube according to a fifth embodiment of the present invention.
Fig. 20 is a cross-sectional view taken along line C-C of Fig. 19.
Fig. 21 is a view explaining an operation of the endoscopic overtube.

### Description of Embodiments

### (First Embodiment)

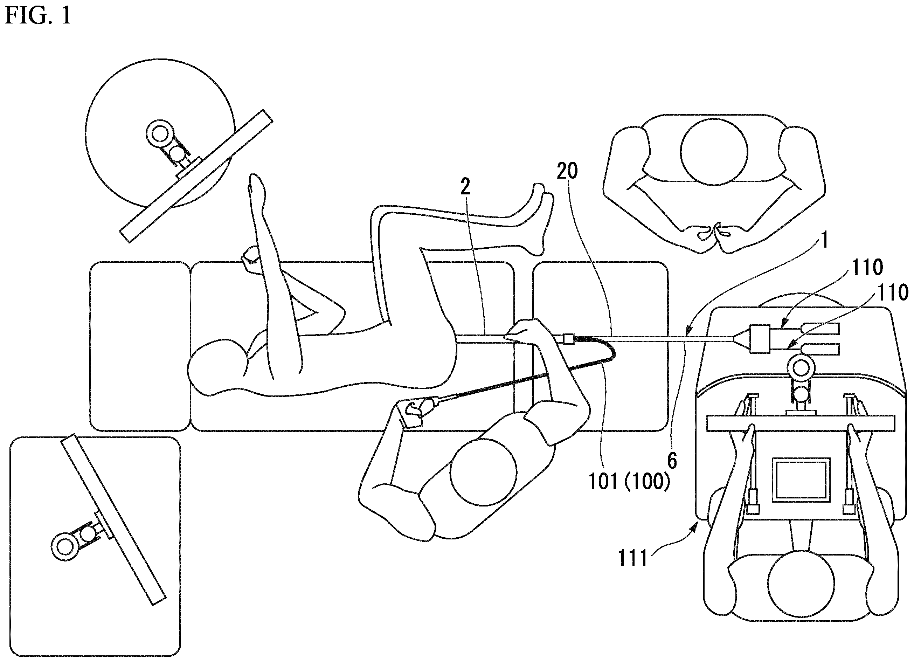

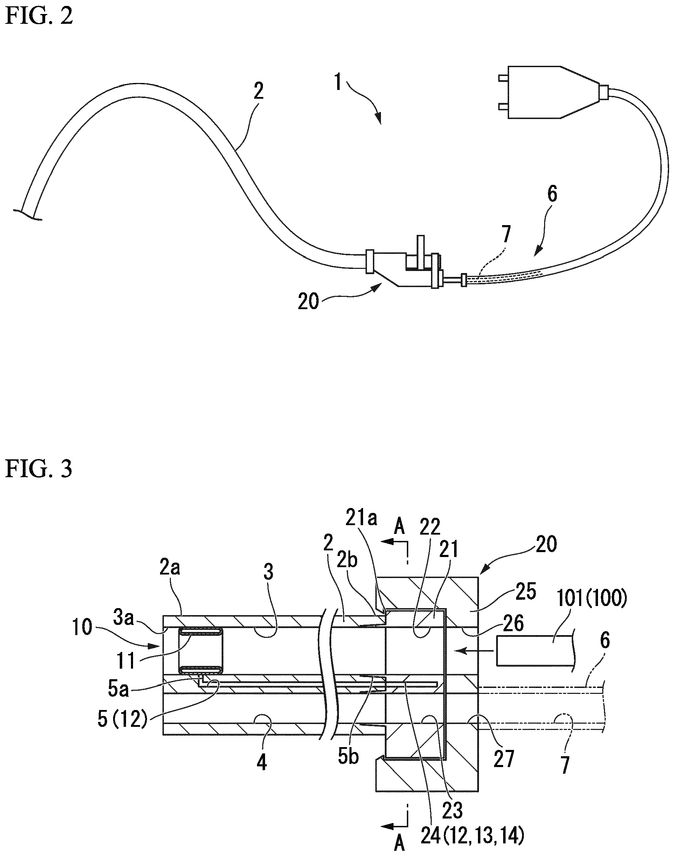

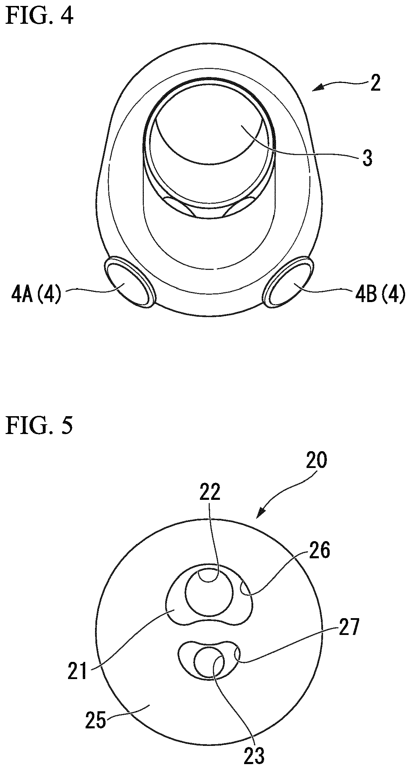

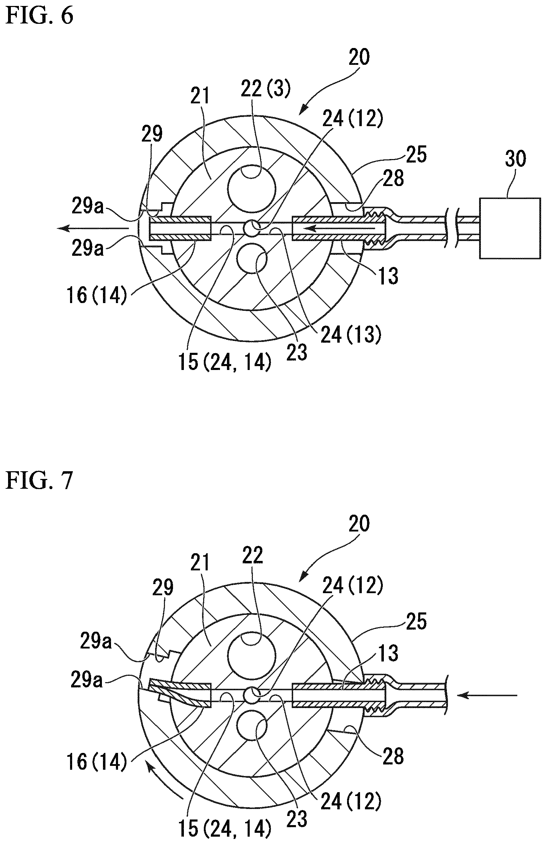

A first embodiment of the present invention will be described. Fig. 1 is an overall view representing a medical system including an endoscopic overtube according to the present embodiment. Fig. 2 is a perspective view of the endoscopic overtube. Fig. 3 is a cross-sectional view of the endoscopic overtube. Fig. 4 is a perspective view of a distal end portion of the endoscopic overtube. Fig. 5 is a rear view of a manipulation part of the endoscopic overtube. Fig. 6 is a cross-sectional view taken along line A-A in Fig. 3.

An endoscopic overtube 1 according to this embodiment represented in Figs. 1 and 2 is an overtube which can be used together with an endoscope 100 having an insertion part 101 to be inserted into a body. The configuration of the endoscope 100 used together with the endoscopic overtube 1 according to the present embodiment is not particularly limited.

The endoscopic overtube 1 includes a tube body 2, a pinching part 10, and a manipulation part 20. Further, the endoscopic overtube 1 according to the present embodiment further includes a treatment tool guide tube 6 which is attachable to and detachable from the manipulation part 20.

As represented in Fig. 3, the tube body 2 is a flexible tubular member in which a first lumen 3, a second lumen 4, and a third lumen 5 are formed. A material of the tube body 2 may be, for example, a material having sufficient hardness to be easily bent to follow a curved shape of a digestive tract when the tube body 2 is inserted into the digestive tract.

The first lumen 3 is a lumen for insertion of the insertion part 101 of the endoscope 100. The first lumen 3 has a center line parallel to a center line of the tube body 2. An inner diameter of the first lumen 3 has a size that is sufficient to freely advance, retract, and rotate the insertion part 101 of the endoscope 100 with respect to the first lumen 3 to correspond to an outer diameter dimension of the insertion part 101 of the endoscope 100 used together with the endoscopic overtube 1.

The second lumen 4 extends to be parallel to the first lumen 3. The second lumen 4 is a lumen for insertion of a medical treatment tool 110 used together with the endoscopic overtube 1 according to the present embodiment.

In the present embodiment, a plurality of second lumens 4 (denoted by reference numerals 4A and 4B in Fig. 4) are provided so that a plurality of medical treatment tools 110 (see Fig. 1) can be inserted therein.

The third lumen 5 is a lumen which constitutes a part of a suction and exhaust conduit 12 through which gas for inflating a balloon 11 to be described later flows. The third lumen 5 has a distal end opening 5a which is open to an inner surface of the first lumen 3 in the vicinity of a distal end 2a of the tube body 2. The distal end opening 5a of the third lumen 5 communicates with an interior of the balloon 11. In the third lumen 5, a proximal end opening 5b, which is an opening opposite to the distal end opening 5a, communicates with a third hole 24 of a fixed member 21 to be described later.

The treatment tool guide tube 6 represented in Figs. 1 to 3 is a flexible or rigid tubular member formed with an extension lumen 7 which communicates with the second lumen 4 of the tube body 2. The treatment tool guide tube 6 extends the second lumen 4 of the tube body 2 up to a manipulator system 111 used at a time of a treatment in which the endoscopic overtube 1 according to the present embodiment is used. Further, the endoscopic overtube 1 according to the present embodiment may not have the treatment tool guide tube 6.

The pinching part 10 represented in Fig. 3 has the balloon 11, the suction and exhaust conduit 12, a supply conduit 13, and a discharge conduit 14.

The balloon 11 is fixed inside the first lumen 3 in the vicinity of the distal end 2a of the tube body 2. The balloon 11 is a hollow bag-shaped elastic member having a part fixed to the inner surface of the first lumen 3, and its shape can be changed. When gas is supplied to the interior of the balloon 11, the balloon 11 is inflatable. When gas inside the inflated balloon 11 is released to the outside, the balloon 11 is in a contracted state.

The balloon 11 can move in a direction in which the balloon 11 approaches the center line of the first lumen 3 by the balloon 11 itself being inflated. Since the balloon 11 is expandable and contractible, a narrow part narrower than the inner diameter of the first lumen 3 can be generated in the first lumen 3, or an annular part can be generated in the first lumen 3 to insert the insertion part 101 of the endoscope 100 with substantially the same diameter as the inner diameter of the first lumen 3 so that the insertion part 101 is freely advanced, retracted and rotated.

The suction and exhaust conduit 12 is a conduit which communicates with the interior of the balloon 11 and extends to the manipulation part 20 along the first lumen 3. The suction and exhaust conduit 12 is constituted of the third lumen 5 formed in the tube body 2, and the third hole 24 formed in the fixed member 21 of the manipulation part 20 to be described later.

The supply conduit 13 is a conduit provided in the manipulation part 20 to be described later to supply gas from an air supply pump 30 for supplying gas to the suction and exhaust conduit 12. The supply conduit 13 communicates with the third hole 24 constituting the suction and exhaust conduit 12 in the fixed member 21 to be described later. The supply conduit 13 protrudes from an outer surface of the manipulation part 20 through a supply side opening portion 28 formed in a rotation member 25 to be described later.

The discharge conduit 14 is a conduit provided in the manipulation part 20 to release gas from the suction and exhaust conduit 12. The discharge conduit 14 communicates with the third hole 24 constituting the suction and exhaust conduit 12 in the fixed member 21 to be described later. The discharge conduit 14 protrudes from the outer surface of the manipulation part 20 through a discharge side opening portion 29 formed in the rotation member 25 to be described later. The discharge conduit 14 has an elastic tubular member 16 fixed to the fixed member 21. In the present embodiment, the discharge conduit 14 is constituted of a branched conduit 15 branching from the third hole 24 formed in the fixed member 21 and the elastic tubular member 16 fixed to the fixed member 21 to communicate with the hole.

The elastic tubular member 16 is an elastic member made of a material which is elastically deformable to be compressed as represented in Fig. 7 by being pinched between an opening end portion of the discharge side opening portion 29 formed in the rotation member 25 described later and the fixed member 21. The elastic tubular member 16 according to the present embodiment is capable of closing an inner cavity of the elastic tubular member 16 by being compressed by relative rotation between the rotation member 25 and the fixed member 21. Further, when relaxing a force of a rotational manipulation of the rotation member 25 with respect to the fixed member 21, the rotation member 25 is pushed back against the fixed member 21 by a restoring force of the elastic tubular member 16 itself, and the inner cavity of the elastic tubular member 16 is restored to an initial open state. When the inner cavity of the elastic tubular member 16 is initially opened, the elastic tubular member 16 opens the discharge conduit 14 so that gas can be discharged from the suction and exhaust conduit 12 and the supply conduit 13 through the discharge conduit 14.

In the present embodiment, the elastic tubular member 16 constituting a part of the discharge conduit 14 functions as a biasing member which biases the rotation member 25 with respect to the fixed member 21 such that a relative position between the fixed member 21 and the rotation member 25 is in a predetermined positional relationship in which the discharge conduit 14 is in an open state.

The pinching part 10 according to the present embodiment provided with the balloon 11, the suction and exhaust conduit 12, the supply conduit 13, and the discharge conduit 14 is configured such that the balloon 11 can come into contact with an outer surface of the insertion part 101 inside the first lumen 3. The pinching part 10 according to the present embodiment can hold the insertion part 101 by the balloon 11 being in contact with the outer surface of the insertion part 101 so that the insertion part 101 is positioned in the first lumen 3.

The manipulation part 20 represented in Figs. 3, 5 and 6 has the fixed member 21 and the rotation member 25.

The fixed member 21 is fixed to a proximal end portion 2b of the tube body 2. A part of the suction and exhaust conduit 12, the supply conduit 13, and the discharge conduit 14 are arranged in the fixed member 21. An annular engaged part 21a formed on an outer circumferential surface to extend along a circumferential direction of the tube body 2 is formed in the fixed member 21. The rotation member 25 can be engaged with the engaged part 21a.

The fixed member 21 has a first hole 22 communicating with the first lumen 3, a second hole 23 communicating with the second lumen 4, and the third hole 24 communicating with the third lumen 5.

The third hole 24 branches in a T shape inside the fixed member 21. The suction and exhaust conduit 12, the supply conduit 13, and the discharge conduit 14 communicate with each other in the fixed member 21 by the third hole 24.

The fixed member 21 may be integrally molded with the tube body 2. The first hole 22 of the fixed member 21 serves as a lumen for insertion of the insertion part 101 of the endoscope 100 together with the first lumen 3 formed in the tube body 2.

The rotation member 25 is a substantially tubular member connected to the fixed member 21 to enable rotational motion with respect to the fixed member 21. The rotation member 25 is connected to the fixed member 21 so as to be engaged with the engaged part 21a of the fixed member 21. Accordingly, the rotation member 25 can rotate with respect to the fixed member 21 about the center line of the tube body 2 as a rotational center. The rotation member 25 according to the present embodiment is connected to the tube body 2 via the fixed member 21. Because the elastic tubular member 16 constituting the discharge conduit 14 comes into contact with the rotation member 25, a rotatable range of the rotation member 25 with respect to the fixed member 21 is limited to a predetermined range. Further, a structure for defining the rotatable range of the rotation member 25 with respect to the fixed member 21 may be provided in the fixed member 21 or the rotation member 25.

The rotation member 25 has a first communication passage 26 and a second communication passage 27 respectively communicating with the first lumen 3 and the second lumen 4, the supply side opening portion 28 formed through an outer wall thereof so that the supply conduit 13 is inserted therethrough, and the discharge side opening portion 29 formed through the outer wall thereof so that the discharge conduit 14 is inserted therethrough.

The first communication passage 26 and the second communication passage 27 have sizes and shapes that do not overlap the first hole 22 and the second hole 23 of the fixed member 21 when viewed from a center line direction of the tube body 2. That is, the first communication passage 26 and the second communication passage 27 are elliptic communication passages having curved shapes which are elongated in the circumferential direction of the tube body 2 corresponding to the rotatable range of the rotation member 25 with respect to the fixed member 21.

As represented in Fig. 6, the supply side opening portion 28 has an elongated hole shape. A size of the supply side opening portion 28 is set such that the rotation member 25 does not come into contact with the supply conduit 13 even if the rotation member 25 rotates within the rotatable range of the rotation member 25 with respect to the fixed member 21.

As represented in Figs. 6 and 7, when the discharge side opening portion 29 is in a positional relationship in which the rotation member 25 rotates with respect to the fixed member 21 with an angle difference equal to or larger than a predetermined angle difference, the discharge side opening portion 29 has a round hole shape or an elongated hole shape having a size such that the discharge side opening portion 29 comes into contact with the elastic tubular member 16. An inner surface of the discharge side opening portion 29 is a pressing surface 29a capable of pressing the elastic tubular member 16 to compress the elastic tubular member 16.

In the present embodiment, the rotation member 25 and the elastic tubular member 16 constitute an exhaust-stopping mechanism which stops exhaust from the discharge conduit 14. The exhaust-stopping mechanism can switch between a state in which the exhaust from the discharge conduit 14 is possible and a state in which exhaust from the discharge conduit 14 is not possible by rotational motion of the rotation member 25 with respect to the fixed member 21. The pressing surface 29a of the rotation member 25 closes the discharge conduit 14 by compressing the elastic tubular member 16 when an angle difference equal to or larger than the predetermined angle difference with respect to the fixed member 21 is generated. As a result, the exhaust-stopping mechanism closes the discharge conduit 14 when there is an angle difference equal to or larger than the predetermined angle difference with respect to the tube body 2, thereby restricting the release of gas from the discharge conduit 14.

As described above, the manipulation part 20 according to the present embodiment can switch between a pinched state in which the insertion part 101 is positioned with respect to the first lumen 3 by the pinching part 10 and an open state in which the insertion part 101 can be advanced, retracted, and rotated inside the first lumen 3 by the rotational motion of the rotation member 25 with respect to the fixed member 21 in the manipulation part 20.

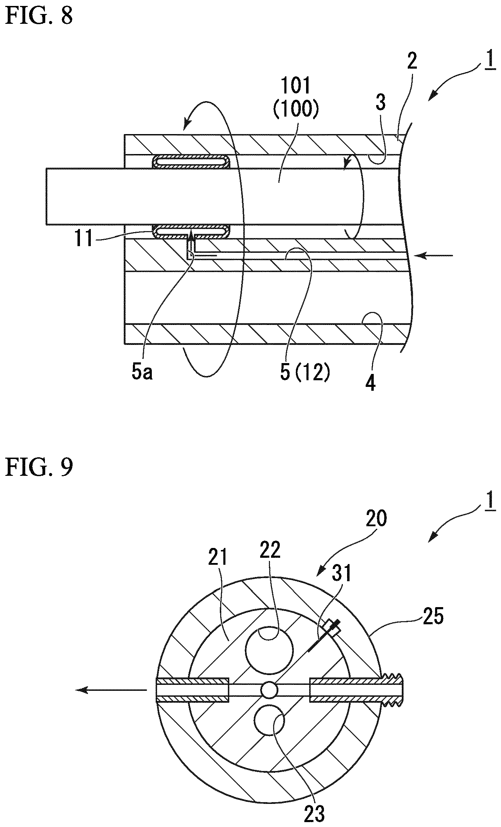

An operation of the endoscopic overtube 1 according to the present embodiment will be described. Figs. 7 and 8 are views explaining an operation of the endoscopic overtube according to this embodiment.

When using the endoscopic overtube 1 according to the present embodiment, as represented in Fig. 6, the air supply pump 30 is attached to the supply conduit 13. A configuration of the air supply pump 30 may be the same as that of a known air supply pump attached to the endoscope 100. When the endoscopic overtube 1 according to the present embodiment is used together with the endoscope 100, in addition to the air supply pump attached to the endoscope 100 and used, another air supply pump 30 is attached to the endoscopic overtube 1.

Further, the endoscopic overtube 1 can be used together with a known medical treatment tool 110 that is suitable for a treatment target. The medical treatment tool 110 attached to the endoscopic overtube 1 and used is inserted into the second lumen 4 of the medical overtube so that treatment can be performed within an imaging field of view of the endoscope 100 inserted into the first lumen 3.

A medical system is configured by combining the endoscopic overtube 1 according to the present embodiment with the endoscope 100, the air supply pump 30, and the medical treatment tool 110. When using this medical system, the air supply pump 30 is always driven and gas is always supplied to the supply conduit 13.

For example, as represented in Fig. 1, the endoscopic overtube 1 is inserted together with the endoscope 100 or before the endoscope 100 into the digestive tract of a patient from a natural opening such as the patient's anus. In general, the endoscope 100 to be inserted into the digestive tract can actively bend a part of the insertion part 101 or can rotate the insertion part 101 around its center line by the endoscope 100 being manipulated to guide the insertion part 101 of the endoscope 100 to a treatment target site through a complicatedly curved digestive tract.

In the endoscopic overtube 1 according to the present embodiment, in some cases, the overtube of the endoscope 100 is required to bend or rotate to follow an active curvature of the insertion part 101 of the endoscope 100, a rotation of the insertion part 101, and the like. Further, in the endoscopic overtube 1, in some cases, the endoscopic overtube 1 is required not to follow the rotation of the insertion part 101 of the endoscope 100, or the endoscopic overtube 1 is required to rotate alone with respect to the endoscope 100 without rotating the insertion part 101 of the endoscope 100.

First, a state in which the insertion part 101 of the endoscope 100 and the endoscopic overtube 1 according to the present embodiment can rotate relative to each other (the open state) will be described.

The open state in the present embodiment is a state in which the balloon 11 (see Fig. 3) does not pinch an outer circumferential surface of the insertion part 101 of the endoscope 100 so that the insertion part 101 of the endoscope 100 is not positioned with respect to the first lumen 3. That is, the open state in the present embodiment is a state in which the balloon 11 is contracted. The state in which the balloon 11 is contracted is a state in which gas is not supplied to the inside of the balloon 11 and gas is released from the balloon 11 to the outside by a contracting force of the balloon 11 itself. The endoscopic overtube 1 is in the open state when a manipulator does not apply a force of operating the rotation member 25 with respect to the fixed member 21.

Next, a state in which the pinching part 10 pinches the insertion part 101 of the endoscope 100 so that the insertion part 101 of the endoscope 100 and the endoscopic overtube 1 according to the present embodiment are integrally rotatable (the pinched state) will be described.

As a manipulation performed by the manipulator to integrally rotate the insertion part 101 of the endoscope 100 and the endoscopic overtube 1 according to the present embodiment, a manipulation of holding both of the insertion part 101 of the endoscope 100 and the manipulation part 20 of the endoscopic overtube 1 and simultaneously rotating both of them (see Fig. 7) is an intuitive manipulation.

In the present embodiment, an inlet of the insertion part 101 of the endoscope 100 with respect to the endoscopic overtube 1 is a part (the first communication passage 26) which opens toward a proximal end portion of the manipulation part 20 of the endoscopic overtube 1 and communicates with the first lumen 3. The manipulator who manipulates the endoscope 100 grasps the insertion part 101 of the endoscope 100 and manipulates to advance, retract, or rotate the insertion part 101. When it is necessary to rotate the endoscopic overtube 1 together with the manipulation part 20, the manipulator grasps a member of the endoscopic overtube 1 located closest to a position at which the manipulator grasps the insertion part 101 of the endoscope 100, that is, the rotation member 25 in this embodiment, together with the insertion part 101 to rotate the endoscopic overtube 1 (see Fig. 1).

When the manipulator rotates the rotation member 25 as represented in Fig. 7, the rotation member 25 rotates with respect to the fixed member 21. At this stage, the tube body 2 does not rotate. As the rotation member 25 rotates with respect to the fixed member 21, the elastic tubular member 16 constituting the discharge conduit 14 is pressed against the pressing surface 29a of the rotation member 25. When the elastic tubular member 16 is compressed, the elastic tubular member 16 is crushed to close the discharge conduit 14.

In a state in which the discharge conduit 14 is closed, as represented in Fig. 8, the gas from the air supply pump 30 which is always in an air supply state is supplied to the suction and exhaust conduit 12 through the supply conduit 13 and is pushed into the balloon 11. Thus, the balloon 11 starts to inflate. When the balloon 11 is inflated, the balloon 11 comes into contact with the outer circumferential surface of the insertion part 101 of the endoscope 100 in the first lumen 3, and further presses the outer circumferential surface of the insertion part 101. When the balloon 11 is inflated, the balloon 11 comes into close contact with the outer circumferential surface of the insertion part 101. Therefore, in a state in which the balloon 11 is inflated, the insertion part 101 of the endoscope 100 is held while being positioned with respect to the first lumen 3. In this manner, the balloon 11 in an inflated state can pinch the insertion part 101 of the endoscope 100.

In the manipulation part 20, in a state in which the manipulator grasps and rotates the insertion part 101 of the endoscope 100 and the rotation member 25 of the endoscopic overtube 1 as represented in Fig. 7, the discharge conduit 14 is continuously closed and the insertion part 101 of the endoscope 100 is rotated by a force transmitted from the manipulator.

As represented in Fig. 8, since the insertion part 101 of the endoscope 100 has a configuration with a high rotational followability of a distal side thereof with respect to a rotational manipulation on a proximal side thereof, the insertion part 101 tends to rotate inside the first lumen 3 in accordance with the manipulation of the manipulator. Here, in the pinched state according to the present embodiment, since the balloon 11 pinches the insertion part 101 of the endoscope 100 in the vicinity of the distal end of the first lumen 3, the distal end 2a of the tube body 2 is also integrally rotated by the distal end of the insertion part 101 being rotated.

That is, in the present embodiment, a force rotating the rotation member 25 by the manipulator is not a force which rotates a part of the distal end 2a of the tube body 2 from a part of the proximal end portion 2b of the tube body 2 through the tube body 2, and the force is used for connecting the insertion part 101 of the endoscope 100 and the tube body 2 in a positioned state at the part of the distal end 2a of the tube body 2. Further, in a force for integrally rotating the insertion part 101 of the endoscope 100 and the endoscopic overtube 1, a force of the manipulator rotating the insertion part 101 of the endoscope 100 is transmitted to a distal end of the endoscopic overtube 1 from a proximal end of the insertion part 101 via a contact part between the distal end of the insertion part 101 and the balloon 11.

When the manipulator stops manipulating the rotation member 25, a blockage of the discharge conduit 14 is eliminated by the restoring force of the elastic tubular member 16. As a result, the endoscopic overtube 1 is brought into the above-mentioned open state and the balloon 11 is contracted so that the insertion part 101 of the endoscope 100 can freely advance, retract, and rotate inside the first lumen 3.

As described above, according to the endoscopic overtube 1 according to the present embodiment, by performing the manipulation on the rotation member 25 of the manipulation part 20 for rotating the endoscopic overtube 1 about the center line of the tube body 2 of the endoscopic overtube 1 as the rotational center thereof, the pinching part 10 (the balloon 11 in the present embodiment) of the endoscopic overtube 1 pinches the outer circumferential surface of the insertion part 101 of the endoscope 100 near the distal end thereof. As a result, by a manipulation of holding the endoscopic overtube 1 and the insertion part 101 of the endoscope 100 with one hand and rotating both of them, the distal end of the insertion part 101 of the endoscope 100 and the distal end of the endoscopic overtube 1 are rotated integrally with each other.

Further, when the manipulator rotates the insertion part 101 of the endoscope 100 without touching the rotation member 25 of the manipulation part 20 of the endoscopic overtube 1, since the pinching part 10 (the balloon 11 in the present embodiment) of the endoscopic overtube 1 does not pinch the outer circumferential surface of the insertion part 101 of the endoscope 100, the insertion part 101 of the endoscope 100 can freely rotate with respect to the first lumen 3 in the first lumen 3 of the endoscopic overtube 1 in accordance with the manipulation of the manipulator.

That is, in the present embodiment, the tube body 2 of the endoscopic overtube 1 may not have a hardness in consideration of the rotational followability of the distal end to the rotation of the proximal end of the tube body 2, and may be, for example, a flexible material that easily deforms to correspond to a curved shape of the digestive tract. The tube body 2 made of such a flexible material can also be integrally rotated together with the insertion part 101 of the endoscope 100 in the present embodiment.

Further, in the present embodiment, after the insertion part 101 of the endoscope 100 and the endoscopic overtube 1 are integrally rotated, only the insertion part 101 is returned to an original state in the open state. Thereby, it is possible to obtain the same result as that of a case of rotating only the endoscopic overtube 1 without rotating the insertion part 101 of the endoscope 100.

Further, in the present embodiment, since the endoscopic overtube is switched to a state of supplying gas into the balloon 11 by closing the elastic tubular member 16 constituting the discharge conduit 14, when it is necessary to inflate the balloon 11, the balloon 11 can be inflated more quickly than a start of driving of the air supply pump 30. A reason for that is that a rise time from starting to drive the air supply pump 30 until the air supply pump 30 enters a state capable of supplying a sufficient amount of gas is not required, the balloon 11 is always slightly pressurized by resistance of gas when the gas supplied from the constantly driven air supply pump 30 escapes from the discharge conduit 14, the interior of the balloon 11 is immediately pressurized due to inertia of the gas flowing from the supply conduit 13 to the discharge conduit 14 when the discharge conduit 14 is closed, and the like.

### (Modified Example 1-1)

A modified example of the aforementioned embodiment will be described. Fig. 9 is a cross-sectional view representing a configuration of the modified example.

As represented in Fig. 9, in this modified example, a spring member 31 (a biasing member) for connecting the fixed member 21 and the rotation member 25 is provided.

The spring member 31 has, for example, a plate shape, and is fixed to the fixed member 21. The spring member 31 fixed to the fixed member 21 can come into contact with the rotation member 25.

When a position at which the elastic tubular member 16 disclosed in the first embodiment is not pressed against the pressing surface 29a of the rotation member 25 is considered as a neutral position, the spring member 31 biases the rotation member 25 against the fixed member 21 to be the neutral position.

Even with such a configuration, the same effect as that of the first embodiment can be obtained.

### (Modified Example 1-2)

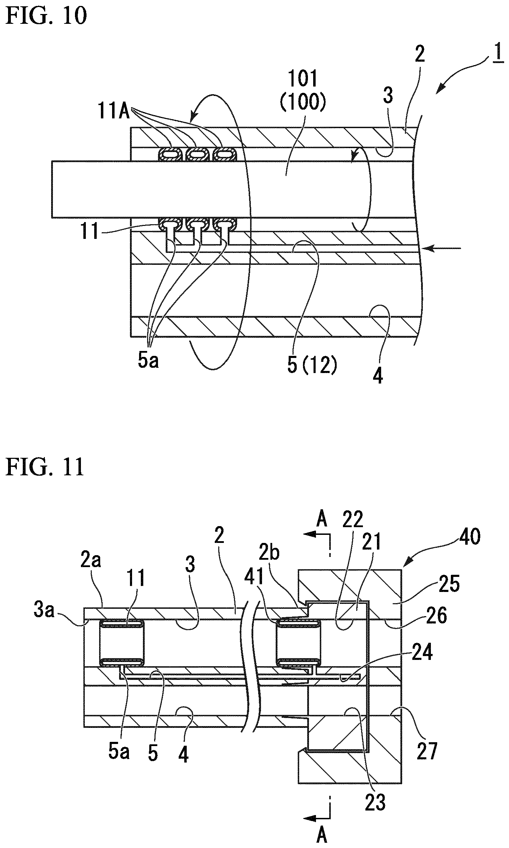

Another modified example of the above embodiment will be described. Fig. 10 is a cross-sectional view representing a configuration of this modified example.

As represented in Fig. 10, in this modified example, the balloon 11 disclosed in the first embodiment is constituted of a plurality of small balloons 11A.

The plurality of small balloons 11A are provided side by side in a center line direction of the first lumen 3. The plurality of small balloons 11A communicate with the third lumen 5. The plurality of small balloons 11A can each be inflated in accordance with the same supply of gas as in the aforementioned first embodiment. In this modified example, expansion and contraction operations of the plurality of small balloons 11A are synchronized with each other.

In this modified example, since the plurality of small balloons 11A are provided side by side, even when the small balloons 11A are inflated, the tube body 2 can be flexibly maintained.

In addition, the small balloons 11A in this modified example may have the same size as that of the balloon 11 in the first embodiment.

### (Second Embodiment)

A second embodiment of the present invention will be described. In each of the embodiments to be described below, the same constituent elements as those disclosed in the first embodiment are denoted by the same reference numerals as in the first embodiment, and repeated description thereof will not be provided. Fig. 11 is a cross-sectional view representing an endoscopic overtube according to this embodiment.

An endoscopic overtube 40 according to this embodiment represented in Fig. 11 has a second balloon 41 at the proximal end portion 2b of the tube body 2.

The second balloon 41 communicates with the third lumen 5 disclosed in the first embodiment. The second balloon 41 can expand and contract in synchronization with the balloon 11 disclosed in the first embodiment. A part or the whole of the second balloon 41 according to the present embodiment is fixed to the first hole 22 of the fixed member 21.

In the present embodiment, when the second balloon 41 is inflated, the insertion part 101 of the endoscope 100 is pinched by the second balloon 41 at a portion of the insertion part 101 of the endoscope 100 located near the proximal end of the tube body 2 of the endoscopic overtube 40. Therefore, in the present embodiment, when a manipulator rotates the rotation member 25 of the manipulation part 20, similarly to the aforementioned first embodiment, the insertion part 101 of the endoscope 100 is pinched by the balloon 11 at the part of the distal end 2a of the tube body 2 and can integrally rotate therewith. Further, unlike the aforementioned first embodiment, since a rotation force to the rotation member 25 is transmitted to the insertion part 101 of the endoscope 100 via the fixed member 21 and the second balloon 41, it is possible to rotate the insertion part 101 of the endoscope 100 with a force for rotating the rotation member 25. In the present embodiment, it is not necessary to grasp and rotate the insertion part 101 and the rotation member 25 at the same time, and the insertion part 101 and the endoscopic overtube 40 can be integrally rotated by rotating the rotation member 25.

For example, when the insertion part 101 of the endoscope 100 is thin, even if grasping and rotating both of the rotation member 25 and the insertion part 101 is intended, there is a possibility that the insertion part 101 slips in a grasping hand. However, in the endoscopic overtube 40 according to the present embodiment, since the insertion part 101 and the endoscopic overtube 40 can be integrally rotated without grasping the insertion part 101, manipulation thereof is simple.

### (Third Embodiment)

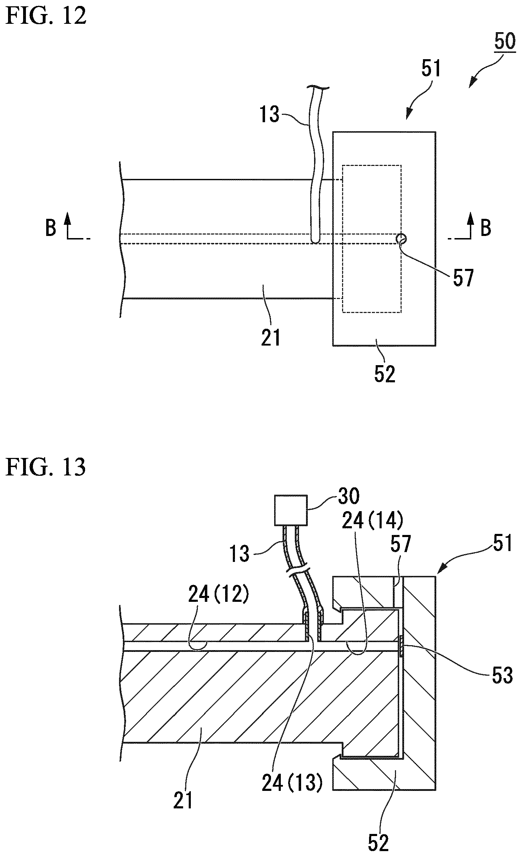

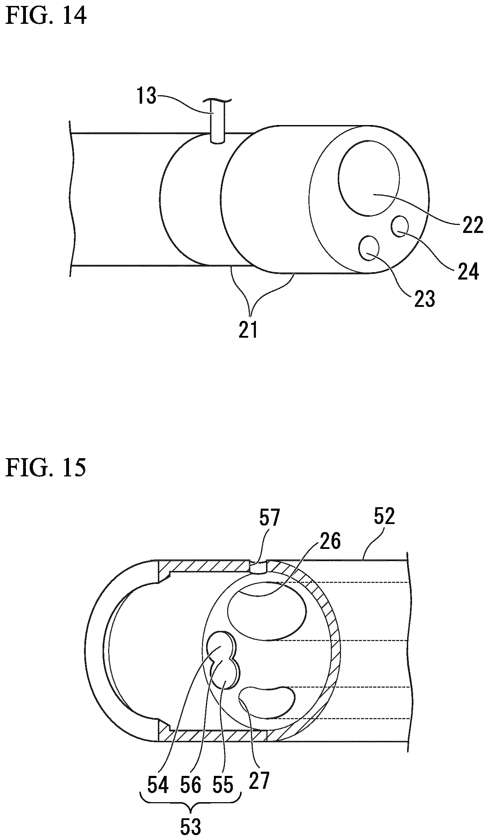

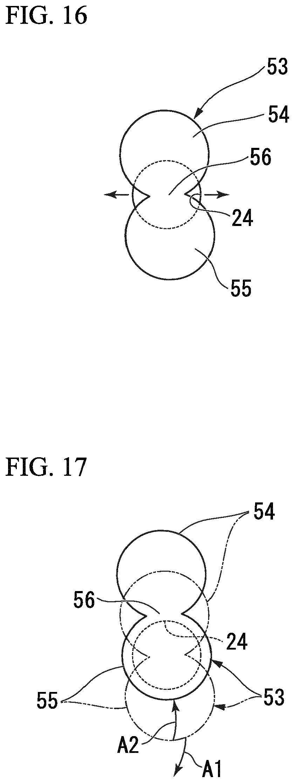

A third embodiment of the present invention will be described. Fig. 12 is a plan view representing an endoscopic overtube according to the present embodiment. Fig. 13 is a cross-sectional view taken along the line B-B of Fig. 12. Fig. 14 is a perspective view representing a part of a manipulation part of the endoscopic overtube. Fig. 15 is a partial cross-sectional view representing a part of the manipulation part of the endoscopic overtube. Fig. 16 is a schematic view explaining an operation of the endoscopic overtube. Fig. 17 is a schematic diagram explaining the operation of the endoscopic overtube.

An endoscopic overtube 50 according to the present embodiment represented in Fig. 12 is provided with a manipulation part 51 which has a configuration different from that of the manipulation part 20 disclosed in the first embodiment in place of the manipulation part 20. Further, in the present embodiment, a discharge conduit 57 having a configuration different from that of the discharge conduit 14 disclosed in the first embodiment is provided in place of the discharge conduit 14.

As represented in Fig. 13, the manipulation part 51 in this embodiment includes the fixed member 21 having the supply conduit 13 formed therein, and a rotation member 52 connected to the fixed member 21 to be rotatable with respect to the fixed member 21.

As represented in Figs. 13 and 15, the rotation member 52 has a lid member 53 which closes the third hole 24 (see Fig. 14) formed in the fixed member 21.

The lid member 53 represented in Figs. 13 and 15 is fixed to the rotation member 52. The lid member 53 closes the third hole 24 of the fixed member 21 when the rotation member 52 is in a predetermined positional relationship with respect to the fixed member 21, to correspond to rotational motion of the rotation member 52 with respect to the fixed member 21. Further, when the rotation member 52 is in another predetermined positional relationship with respect to the fixed member 21, the lid member 53 allows gas to be released from the third hole 24 of the fixed member 21. In the present embodiment, the gas released from the third hole 24 is released to the outside of the manipulation part 51 via the discharge conduit 57 through a gap between the fixed member 21 and the rotation member 52.

Further, in the present embodiment, the rotation member 52 is biased with respect to the fixed member 21 to a predetermined neutral position by, for example, a biasing member (not represented) similar to the biasing member (e.g., the spring member 31) disclosed in the modified example of the first embodiment.

The predetermined positional relationship in which the lid member 53 closes the third hole 24 is a positional relationship (see Fig. 17) in which the rotation member 52 is rotated with respect to the fixed member 21 at a predetermined angle difference from the neutral position (see Fig. 16). The predetermined positional relationship represented in Fig. 17 includes a first position corresponding to an angle difference rotated in a clockwise direction (indicated by reference numeral A1 in Fig. 17) when seen from a proximal end side to a distal end side of the endoscopic overtube 50, and a second position corresponding to an angle difference rotated in a counterclockwise direction (indicated by reference character A2 in Fig. 17).

As represented in Figs. 15 to 17, a shape of the lid member 53 is a substantial figure eight which has a first large-diameter region 54 having a circular shape with a larger diameter than an inner diameter of the third hole 24 when the rotation member 52 is located at the first position, a second large-diameter region 55 having a circular shape with a larger diameter than the inner diameter of the third hole 24 when the rotation member 52 is located at the second position, and a small-diameter region 56 which connects the first large-diameter region 54 and the second large-diameter region 55. The small-diameter region 56 is a region which does not restrict the discharge of gas from the third hole 24 by having a shape which blocks only a part of the third hole 24. Since the first large-diameter region 54 and the second large-diameter region 55 of the lid member 53 are connected to each other by the small-diameter region 56, the lid member 53 can move without being caught by the third hole 24 when the rotation member 52 rotates with respect to the fixed member 21.

The lid member 53 is, for example, made of a resin such as rubber so as to come into contact with the third hole 24 in an airtight state.

The endoscopic overtube 50 according to the present embodiment also has the same effects as those of the first embodiment.

### (Fourth Embodiment)

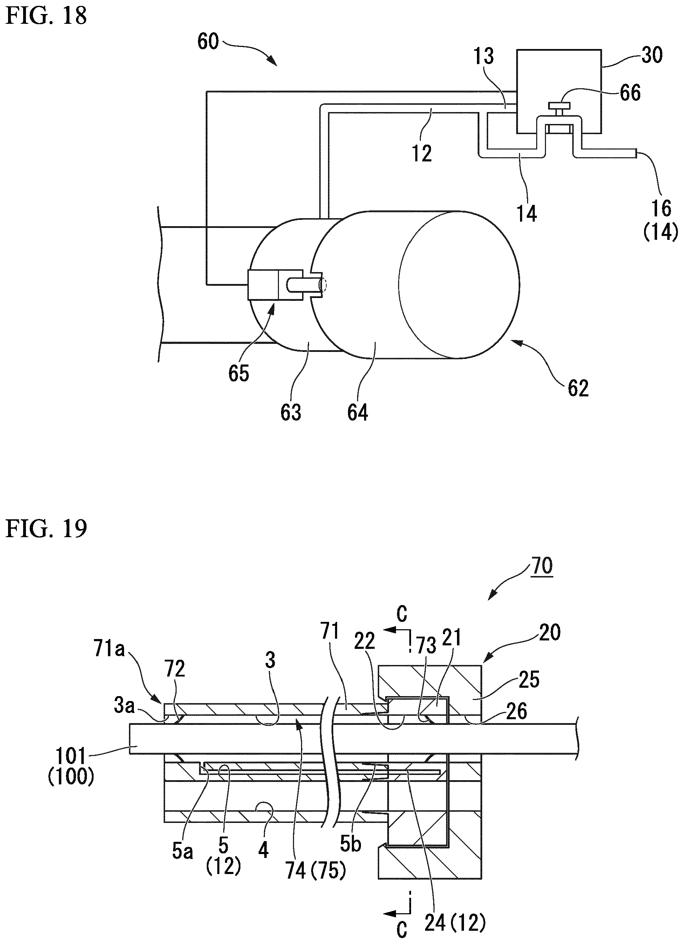

A fourth embodiment of the present invention will be described. Fig. 18 is a perspective view representing an endoscopic overtube according to this embodiment.

An endoscopic overtube 60 according to the present embodiment represented in Fig. 18 is provided with a pinching part 61 having a configuration different from that of the pinching part 10 disclosed in the first embodiment in place of the pinching part 10. Further, the endoscopic overtube 60 according to the present embodiment is provided with a manipulation part 62 having a configuration different from that of the manipulation part 20 disclosed in the first embodiment in place of the manipulation part 20.

In the pinching part 61, the suction and exhaust conduit 12 disclosed in the first embodiment is drawn from the manipulation part 62 to the external air supply pump 30. The supply conduit 13 and the discharge conduit 14 are arranged outside the manipulation part 62.

Further, in the present embodiment, there is a solenoid 66 which presses the elastic tubular member 16 constituting the discharge conduit 14 to close the discharge conduit 14 as an exhaust-stopping mechanism.

The manipulation part 62 has a fixed member 63 and a rotation member 64 which can rotate relative to each other, like the fixed member 21 disclosed in the first embodiment and the rotation member 52, and a switch 65 which operates the solenoid 66 when there is a positional relationship in which the rotation member 52 is rotated at a predetermined angle difference from a neutral position with respect to the fixed member 21. The switch 65 is a switch which switches turning on-off a signal for driving the solenoid 66. The switch 65 is pushed and turned on when there is the positional relationship in which the rotation member 52 is rotated at the predetermined angle difference from the neutral position with respect to the fixed member 21.

In the present embodiment, the elastic tubular member 16 is compressed by the solenoid 66 while the switch 65 operates the solenoid 66 so that the discharge conduit 14 is in a closed state.

The endoscopic overtube 60 of the present embodiment also has the same effects as those of the first embodiment.

### (Fifth Embodiment)

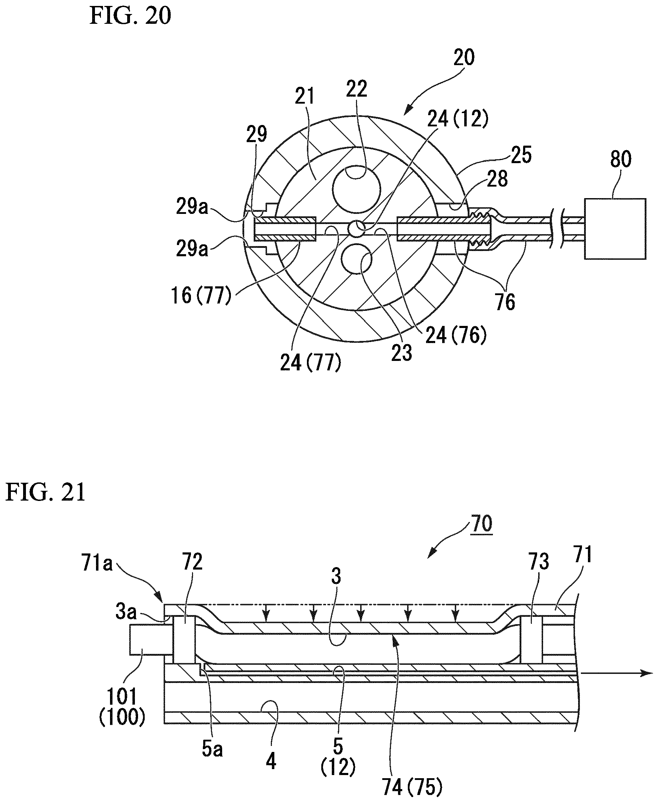

A fifth embodiment of the present invention will be described. Fig. 19 is a cross-sectional view representing an endoscopic overtube according to this embodiment. Fig. 20 is a cross-sectional view taken along line C-C of Fig. 19. Fig. 21 is a view explaining an operation of the endoscopic overtube.

An endoscopic overtube 70 according to the present embodiment represented in Figs. 19 and 20 is an overtube which can be used together with the endoscope 100 having the insertion part 101 to be inserted into a body. The configuration of the endoscope 100 used together with the endoscopic overtube 70 according to the present embodiment is not particularly limited, but it is preferable to use the endoscope 100 provided with an insertion part 101 having an outer diameter corresponding to the inner diameter of the first lumen 3 to be described later.

The endoscopic overtube 70 includes a tube body 71, a pinching part 75, and the manipulation part 20.

The tube body 71 is a flexible tubular member in which the first lumen 3, the second lumen 4, and the third lumen 5 are formed. A material of the tube body 71 may be, for example, a material having sufficient hardness to be easily bent to follow a curved shape of a digestive tract when the tube body 71 is inserted into the digestive tract. Further, the tube body 71 according to the present embodiment has a configuration flexible enough to be deformed such that the first lumen 3 decreases in diameter by a negative pressure when an interior of the first lumen 3 is in a negative pressure state.

The first lumen 3 is a lumen having a center line which is parallel to a center line of the tube body 71. The first lumen 3 is a lumen for insertion of the insertion part 101 of the endoscope 100. The inner diameter of the first lumen 3 is set to such a size that the insertion part 101 of the endoscope 100 can be freely advanced, retracted, and rotated with respect to the first lumen 3 to correspond to an outer diameter size of the insertion part 101 of the endoscope 100 to be used together with the endoscopic overtube 70 according to the present embodiment.

An annular first airtight sealing part 72 protruding from the inner surface of the first lumen 3 toward the center line direction of the first lumen 3 is formed on an inner circumferential surface of the distal end portion 3a of the first lumen 3. An inner diameter of the first airtight sealing part 72 is smaller than the outer diameter of the insertion part 101 of the endoscope 100 which becomes a target to be attached to the endoscopic overtube 70 according to the present embodiment. That is, when the insertion part 101 of the endoscope 100 is inserted into the first airtight sealing part 72, the first airtight sealing part 72 separates a region generated between the inner surface of the first lumen 3 and the outer circumferential surface of the insertion part 101 of the endoscope 100 into two regions of a proximal end side and a distal end side of the first airtight sealing part 72 while maintaining the airtight state.

An annular second airtight sealing part 73 protruding in a direction from the inner surface of the first lumen 3 toward the center line of the first lumen 3 is formed at a position spaced apart toward the proximal end side from the first airtight sealing part 72 in the center line direction of the first lumen 3. The second airtight sealing part 73 according to the present embodiment is provided in the first hole 22 of the fixed member 21. An inner diameter of the second airtight sealing part 73 is smaller than the outer diameter of the insertion part 101 of the endoscope 100 which becomes a target to be attached to the endoscopic overtube 70 according to the present embodiment. That is, when the insertion part 101 of the endoscope 100 is inserted into the second airtight sealing part 73, the second airtight sealing part 73 separates a region generated between the inner surface of the first lumen 3 and the outer circumferential surface of the insertion part 101 into two regions of a proximal end side and a distal end side of the second airtight sealing part 73 while maintaining the airtight state.

In a state in which the insertion part 101 is inserted into both of the first airtight sealing part 72 and the second airtight sealing part 73, a space partitioned in the airtight state toward the outside is generated by the first airtight sealing part 72, the second airtight sealing part 73, the inner circumferential surface of the first lumen 3, and the outer circumferential surface of the insertion part 101. Hereinafter, the partitioned region which defines the space in the tube body 71 is referred to as a pinching region 74. The inner surface of the first lumen 3 which is located between the first airtight sealing part 72 and the second airtight sealing part 73 positions and holds the insertion part 101 of the endoscope 100 with respect to the first lumen 3, like the balloon 11 disclosed in the first embodiment, by pinching the outer surface of the insertion part 101 when the space defined by the pinching region 74 is depressurized, which will be described in detail later.

The second lumen 4 extends to be parallel to the first lumen 3. The second lumen 4 is a lumen for insertion of the medical treatment tool 110 which is used together with the endoscopic overtube 70 according to the present embodiment.

The third lumen 5 constitutes a part of the suction and exhaust conduit 12 through which gas flows to depressurize the space defined by the pinching region 74. The third lumen 5 has the distal end opening 5a which opens toward an inner circumferential surface between the first airtight sealing part 72 and the second airtight sealing part 73 of the first lumen 3. In the present embodiment, the third lumen 5 has the distal end opening 5a at a position slightly separated from the proximal end side of the first airtight sealing part 72 in the vicinity of a distal end 71a of the tube body 71. When the distal end opening 5a of the third lumen 5 is in the vicinity of the distal end portion 3a of the first lumen 3, it is possible to discharge gas in the pinching region 74 from a distal end side of the pinching region 74. Further, the position of the distal end opening 5a of the third lumen 5 may be separated from the first airtight sealing part 72 toward the proximal end side thereof, or the distal end opening 5a may be in the vicinity of the second airtight sealing part 73. The proximal end opening 5b, which is an opening on the opposite side of the distal end opening 5a of the third lumen 5, communicates with the third hole 24 of the fixed member 21 to be described later.

The pinching part 75 according to the present embodiment has the pinching region 74 configured in the first lumen 3, the suction and exhaust conduit 12, a suction conduit 76, and an inflow conduit 77.

The suction and exhaust conduit 12 has the same configuration as that of the first embodiment, but unlike the first embodiment, gas flows to depressurize the space defined by the pinching region 74 by suction, and when the suction is released in a decompressed state, gas flowing into the space flows conversely by a restoring force of the tube body 71.

Although the suction conduit 76 has the same configuration as the supply conduit 13 disclosed in the first embodiment, it differs from the supply conduit 13 in that a suction pump 80 is attached in place of the air supply pump 30 being attached. The suction pump 80 always suctions air when the endoscopic overtube 70 is used.

The inflow conduit 77 has the same configuration as the discharge conduit 14 disclosed in the first embodiment, but in contrast to the first embodiment, the gas is made to flow in from the outside by the gas being suctioned from the suction conduit 76 by the suction pump 80. The inflow conduit 77 according to the present embodiment has the elastic tubular member 16 disclosed in the first embodiment. The elastic tubular member 16 can be compressed by the rotation of the rotation member 25 with respect to the fixed member 21 as in the first embodiment. Compression of the elastic tubular member 16 by the rotation member 25 closes the inflow conduit 77.

In the present embodiment, the elastic tubular member 16 constituting a part of the inflow conduit 77 functions as a biasing member which biases the rotation member 25 with respect to the fixed member 21 such that a relative position between the fixed member 21 and the rotation member 25 becomes a predetermined positional relationship in which the inflow conduit 77 is in the open state.

In the pinching part 75 according to the present embodiment equipped with the pinching region 74 (including the first airtight sealing part 72 and the second airtight sealing section 73), the suction and exhaust conduit 12, the suction conduit 76, and the inflow conduit 77, an inner circumferential surface of the pinching region 74 can come into contact with the outer surface of the insertion part 101 in the first lumen 3. The pinching part 75 according to the present embodiment can hold the insertion part 101 by the inner circumferential surface of the first lumen 3 being in contact with the outer surface of the insertion part 101 so that the insertion part 101 is positioned in the first lumen 3. In the present embodiment, a shape of the pinching part 75 can be changed by suction through the suction and exhaust conduit 12.

The manipulation part 20 has the fixed member 21 and the rotation member 25 disclosed in the first embodiment. The manipulation part 20 differs from the first embodiment only in that the suction conduit 76 and the inflow conduit 77 are disposed in place of the supply conduit 13 and the exhaust conduit disclosed in the first embodiment, and other configurations are similar to those of the first embodiment.

In the present embodiment, the rotation member 25 and the elastic tubular member 16 constitute an inflow-stopping mechanism which stops the inflow of the gas from the inflow conduit 77. The inflow-stopping mechanism can switch between a state in which the inflow of outside air from the inflow conduit 77 is possible and a state in which the inflow of the outside air from the inflow conduit 77 is not possible by rotational motion of the rotation member 25 with respect to the fixed member 21. The pressing surface 29a of the rotation member 25 closes the inflow conduit 77 by compressing the elastic tubular member 16 when an angle difference equal to or larger than a predetermined angle difference with respect to the fixed member 21 is generated. As a result, the inflow-stopping mechanism closes the inflow conduit 77 when an angle difference equal to or greater than the predetermined angle difference with respect to the tube body 71 is generated, restricts the inflow of gas from the inflow conduit 77, and starts decompressing the space defined by the pinching region 74.

As described above, the manipulation part 20 according to the present embodiment can switch between a pinched state in which the insertion part 101 is positioned with respect to the first lumen 3 by the pinching part 75, and an open state in which the insertion part 101 can advance, retract, and rotate inside the first lumen 3, by the rotational motion of the rotation member 25 with respect to the fixed member 21 in the manipulation part 20.

An operation of the endoscopic overtube 70 according to the present embodiment will be described.

When the endoscopic overtube 70 according to the embodiment represented in Fig. 19 is used, the suction pump 80 is attached to the suction conduit 76 as represented in Fig. 20. A configuration of the suction pump 80 may be the same as that of a known suction pump attached to the endoscope 100. Further, the suction pump 80 may be configured to trap bodily fluids and the like entering the first lumen 3. When the endoscopic overtube 70 according to the present embodiment is used together with the endoscope 100, in addition to the suction pump attached to the endoscope 100 and used, another suction pump 80 is attached to the endoscopic overtube 70.

Further, the endoscopic overtube 70 can be used together with the medical treatment tool 110 as in the first embodiment.

A medical system is configured by combining the endoscopic overtube 70 according to the present embodiment with the endoscope 100, the suction pump 80, and the medical treatment tool 110. When this medical system is used, the suction pump 80 is always driven, and gas is always suctioned from the suction conduit 76.

A state (the open state) in which the insertion part 101 of the endoscope 100 and the endoscopic overtube 70 according to the present embodiment can rotate relative to each other will be described.

The open state in the present embodiment is a state in which the space defined by the pinching region 74 is normal pressure (atmospheric pressure) and the inner circumferential surface of the first lumen 3 does not pinch the outer circumferential surface of the insertion part 101 so that the insertion part 101 is not positioned with respect to the first lumen 3 (see Fig. 19). That is, the open state in the present embodiment is a state in which the first lumen 3 maintains an initial inner diameter larger than the outer diameter of the insertion part 101 by the restoring force of the tube body 71 itself.

Next, a state (the pinched state) in which the pinching part 75 pinches the insertion part 101 so that the insertion part 101 of the endoscope 100 and the endoscopic overtube 70 according to the present embodiment can rotate integrally will be described.

A manipulation performed by a manipulator to integrally rotate the insertion part 101 of the endoscope 100 and the endoscopic overtube 70 according to the present embodiment is an intuitive manipulation performed by the manipulator as in the first embodiment.

When the manipulator grasps and rotates the rotation member 25 represented in Fig. 20 with respect to the fixed member 21, the rotation member 25 rotates with respect to the fixed member 21. At this stage, the tube body 71 does not rotate. As the rotation member 25 rotates with respect to the fixed member 21, the elastic tubular member 16 constituting the inflow conduit 77 is compressed by the pressing surface 29a of the rotation member 25. When the elastic tubular member 16 is compressed, the elastic tubular member 16 is crushed to close the inflow conduit 77.

When the inflow conduit 77 is closed, the suction conduit 76, the suction and exhaust conduit 12, and an interior space of the pinching region 74 are depressurized by the suction pump 80 which is always in the suction state. As represented in Fig. 21, when the space defined by the pinching region 74 is depressurized, the tube body 71 is elastically deformed so that the inner surface of the first lumen 3 comes into contact with the outer circumferential surface of the insertion part 101 of the endoscope 100. Thus, the insertion part 101 of the endoscope 100 is held in a state of being positioned with respect to the first lumen 3. In this manner, the pinching region 74 in the depressurized state can pinch the insertion part 101 of the endoscope 100.

When the manipulator stops the manipulation of the rotation member 25 represented in Fig. 20, blockage of the inflow conduit 77 is eliminated by the restoring force of the elastic tubular member 16. As a result, the endoscopic overtube 70 is in the open state and the tube body 71 is restored to its initial shape such that the insertion part 101 of the endoscope 100 can freely advance, retract, and rotate inside the first lumen 3.

As described above, according to the endoscopic overtube 70 according to the present embodiment, by the manipulation for rotating the endoscopic overtube 70 about the center line of the tube body 71 of the endoscopic overtube 70 as a rotational center being performed on the rotation member 25 of the manipulation part 20, the pinching part 75 (the inner surface of the first lumen 3 in the pinching region 74 in the present embodiment) of the endoscopic overtube 70 holds the outer circumferential surface near the distal end of the insertion part 101 of the endoscope 100. As a result, by a manipulation of grasping the endoscopic overtube 70 and the insertion part 101 of the endoscope 100 with one hand and rotating both of them, the distal end of the insertion part 101 of the endoscope 100 and a distal end of the endoscopic overtube 70 rotate integrally with each other.

Although the embodiments of the present invention have been described in detail with reference to the drawings, the specific configuration is not limited to the embodiments, and design changes and the like within a scope that does not depart from the gist of the present invention are included.

Further, the constituent elements described in each of the embodiments and the modified examples can be configured by appropriately combining them.

### Industrial Applicability

The present invention can be applied to an endoscopic overtube and a medical system.

### Reference Signs List

1, 40, 50, 60, 70: Endoscopic overtube
2, 71: Tube body
3: First lumen
4: Second lumen
5: Third lumen
6: Treatment tool guide tube
7: Extension lumen
10, 61, 75: Pinching part
11: Balloon
11A: Small balloon
12: Suction and exhaust conduit
13: Supply conduit
14, 57: Discharge conduit
15: Branched conduit
16: Elastic tubular member
20, 51, 62: Manipulation part
21, 63: Fixed member
21a: Engaged part (groove)
22: First hole
23: Second hole
24: Third hole
25, 52, 64: Rotation member
26: First communication passage
27: Second communication passage
28: Supply side opening portion
29: Discharge side opening portion
29a: Pressing surface
30: Air supply pump
31: Spring material
41: Second balloon
53: Lid member
54: First large-diameter region
55: Second large-diameter region
56: Small-diameter region
65: Switch
66: Solenoid
72: First airtight sealing part
73: Second airtight sealing part
74: pinching region
76: Suction conduit
77: Inflow conduit
80: Suction pump
100: Endoscope
101: Insertion part
110: Medical treatment tool

## Claims

1. An endoscopic overtube which is usable with an endoscope having an insertion part to be inserted into a body, the endoscopic overtube comprising:
a tube body in which a first lumen for insertion of the insertion part is formed;
a shape-changeable pinching part which is capable of coming into contact with an outer surface of the insertion part inside the first lumen, the pinching part being capable of holding the insertion part by coming into contact with the outer surface of the insertion part such that the insertion part is positioned in the first lumen; and
a manipulation part which is connected to an end portion of the tube body such that the manipulation part is capable of performing rotational motion of rotating with respect to the tube body about a line parallel to a center line of the first lumen as a rotational center thereof, the manipulation part switching by the rotational motion between a pinched state in which the insertion part is positioned with respect to the first lumen by the pinching part and an open state in which the insertion part is capable of advancing, retracting, and rotating in the first lumen.

2. The endoscopic overtube according to claim 1, wherein the pinching part includes:
a balloon which is capable of expanding and contracting, the balloon being fixed to the first lumen;
a suction and exhaust conduit which communicates with an inside of the balloon, the suction and exhaust conduit extending to the manipulation part along the first lumen;
a supply conduit which is provided in the manipulation part and communicates with the suction and exhaust conduit to supply gas from a pump for supplying the gas to the suction and exhaust conduit; and
a discharge conduit which is provided in the manipulation part and communicates with the suction and exhaust conduit to release the gas from the suction and exhaust conduit,
wherein the manipulation part includes an exhaust-stopping mechanism which closes the discharge conduit to restrict a release of the gas from the discharge conduit when a rotation angle difference with respect to the tube body becomes equal to or greater than a predetermined angle difference.

3. The endoscopic overtube according to claim 2, wherein the manipulation part has:
a fixed member in which at least a part of each of the suction and exhaust conduit, the supply conduit, and the discharge conduit is disposed, the fixed member being fixed to the end portion of the tube body;
a rotation member which has the exhaust-stopping mechanism, the rotation member being connected to the fixed member such that the rotation member is capable of performing the rotational motion with respect to the fixed member; and
a biasing member which biases the rotation member with respect to the fixed member such that a relative position between the fixed member and the rotation member is in a predetermined positional relationship in which the discharge conduit is in an open state.

4. The endoscopic overtube according to claim 3, wherein the biasing member has an elastic tubular member which is made of an elastic member and constitutes the discharge conduit, and
the rotation member has a pressing surface which closes the discharge conduit by compressing the elastic tubular member when an angle difference with respect to the fixed member equal to or larger than the predetermined angle difference is generated.

5. The endoscopic overtube according to claim 3, wherein the biasing member is made of a spring material which connects the fixed member and the rotation member.

6. The endoscopic overtube according to claim 2, wherein the balloon is disposed on an end side opposite to the end portion of the tube body.

7. The endoscopic overtube according to claim 6, wherein the balloon has a second balloon disposed at the end portion of the tube body.

8. The endoscopic overtube according to claim 1, wherein the pinching part includes:
an annular first airtight sealing part which is disposed at an end opposite to the end portion of the tube body in a center line direction of the tube body, the first airtight sealing part being capable of coming into contact with an outer circumferential surface of the insertion part over an entire circumference thereof;
an annular second airtight sealing part which is disposed at the end portion of the tube body in the center line direction, the second airtight sealing part being capable of coming into contact with the outer circumferential surface of the insertion part over the entire circumference;
a suction conduit which is opened to an inner surface of the first lumen between the first airtight sealing part and the second airtight sealing part, the suction conduit being connectable to a pump which suctions gas; and
an outside air introduction conduit which is connected to the suction conduit at a part of an intermediate portion of the suction conduit,
wherein the manipulation part includes an inflow-stopping mechanism which closes the outside air introduction conduit to restrict inflow of the gas from the outside air introduction conduit when a rotation angle difference with respect to the tube body equal to or larger than a predetermined angle difference is generated.

9. The endoscopic overtube according to any one of claims 1 to 8, wherein the tube body has a second lumen extending to be parallel to the first lumen.

10. A medical system comprising:
the endoscopic overtube according to any one of claims 2 to 8;
an endoscope having an insertion part which is insertable into the first lumen; and
the pump.
